# EUROPEAN PATENT SPECIFICATION

(11) **EP 1 887 943 B1**
(45) Date of publication and mention of the grant of the patent: **28.10.2015**
(21) Application number: 06740886.4
(22) Date of filing: 12.04.2006
(51) Int. Cl.: A61B 17/04

(54) **APPARATUS FOR SURGICAL REPAIR**
GERÄT FÜR CHIRURGISCHE REPARATUR
APPAREIL DE REPARATION CHIRURGICALE

(30) Priority: 20.04.2005 US 110540; 20.04.2005 US 110584; 20.04.2005 US 110004
(43) Date of publication of application: 20.02.2008
(73) Proprietor: Arthroscopic Innovations LLC, Weymouth, MA 02188 (US)
(72) Inventor: CERUNDOLO, Daniel Arthroscopic Innovations LLC, Weymouth, MA 02188 (US)
(74) Representative: Grey, Ian Michael
(86) International application number: PCT/US2006/013617
(87) International publication number: WO 2006/115782

(56) References cited:
- WO-A-03/007799
- US-A1- 2002 133 165
- US-A1- 2004 193 172
- US-A1- 2004 199 166

## Description

This invention relates to apparatus for surgical repair.

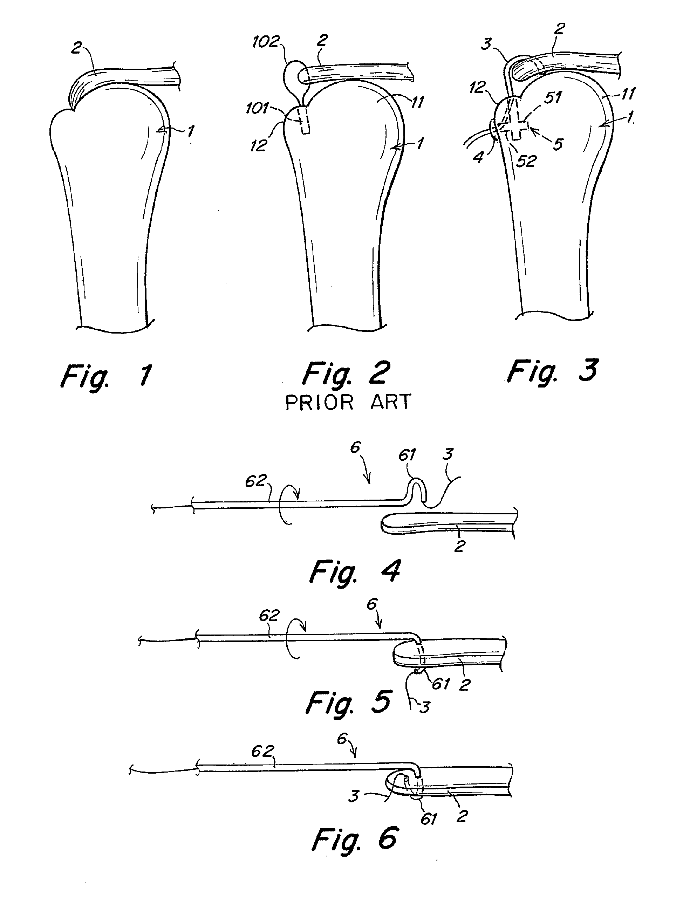

Suture anchors and other suture fixation devices are often used for surgical repair, such as when attempting to secure one body portion relative to another or relative to a surgical implant or other device. For example, tendon damage frequently requires surgery for repair, e.g., to reattach a torn or separated tendon to the bone to which the tendon would normally be attached. Shoulder rotator cuff injuries typically involve damage to the rotator cuff tendon such that the tendon, or at least a portion thereof, requires reattachment to the humerus. Figure 1 shows a schematic diagram of a humerus 1 and a portion of a rotator cuff tendon 2 that is normally attached to the head of the humerus. In one type of damage to the rotator cuff, the tendon 2 may detach or be partially torn from the humerus 1, such as that shown schematically in Figure 2. Such damage may be repaired by reattaching the rotator cuff tendon to the humerus 1 by a suture or other fixation so that the body's normal healing processes can naturally effect reattachment of the tendon to the bone. One repair technique for reattaching the rotator cuff 2 to the humerus 1 involves fixing an anchor 101 at a margin between the articulating portion 11 of the humerus 1 and the humerus' greater tuberosity 12. A suture 102 is secured to the rotator cuff 2 and the anchor 101, and the suture 102 is tensioned so that the rotator cuff 2 is held in place close to the humerus 1. Thereafter, the body may reestablish the proper attachment of the rotator cuff 2 to the humerus 1.

Apparatus for use with a passageway in a surgical procedure having the features recited in the pre-characterising part of claim 1 is known from US2002/0133165 A1.

Apparatus according to the present invention is characterised by the features recited in the characterising portion of claim 1.

Preferred features of the invention are defined in the dependent claims.

Various aspects of the invention are described with reference to illustrative embodiments, wherein like numerals reference like elements, and wherein:
FIGURE 1 is a schematic diagram of a head of a humerus and attached rotator cuff tendon;
FIGURE 2 shows a prior art technique for repairing a rotator cuff injury;
FIGURE 3 is a schematic diagram of a tissue repair arrangement in accordance with an aspect of the invention;
FIGS. 4 - 6 show the use of a needle for placing a suture in a tissue in accordance with the invention;
FIGURE 7 shows a needle in engagement with a cannula in accordance with an aspect of the invention;
FIGURE 8 shows an illustrative arrangement for engaging a needle with a cannula in one embodiment;
FIGURE 9 shows an illustrative arrangement for engaging a sleeve and needle assembly with a cannula in accordance with another embodiment;
FIGURE 10 shows a guide apparatus used in forming a passageway in accordance with the invention;
FIGURE 11 shows the use of a guide apparatus for passing a suture or other element through a transosseous passageway in accordance with the invention;
FIGURE 12 shows a technique for passing a suture placed in a tissue through a passageway;
FIGURE 13 shows the engagement of a suture with a suture fixation device in accordance with the invention;
FIGURE 14 shows the placement of a suture fixation device relative to the bone in accordance with the invention;
FIGURE 15 shows a suture fixation device engagement tool in engagement with a suture fixation device in accordance with the invention; and
FIGS. 16A-B and 17A-B show side and rear views, respectively, of illustrative embodiments of suture fixation devices in accordance with the invention.

This invention is not limited in its application to the details of construction and the arrangement of components set forth in the following description or illustrated in the drawings. The invention is capable of other embodiments and of being practiced or of being carried out in various ways. Also, the phraseology and terminology used herein is for the purpose of description and should not be regarded as limiting. Also, the term "guide" or "guiding" as used herein means actually guiding an element in its movement, and/or providing some reference for the location of an element. For example, "guiding" the formation of a hole in a body portion may mean guiding a bone perforator when actually forming the hole and/or providing a starting location for the hole formation. Various aspects of the invention are described below with reference to specific embodiments. For example, aspects of the invention are described in the context of performing a rotator cuff repair. However, it should be understood that aspects of the invention are not necessarily restricted to rotator cuff repair techniques, or even to surgical techniques performed on a shoulder. Rather, various aspects of the invention may be used in any suitable surgical procedure. In addition, various aspects of the invention may be used alone, and/or in combination with any other aspects of the invention.

As an example, a method for performing a surgical procedure may include providing a passageway in a body portion where the passageway extends from a first opening in the body portion, e.g., a bone, to a second opening in the body portion.

A suture may be placed in or otherwise secured to a material, e.g., a tissue, prosthetic, surgical implant, etc., to be secured relative to the body portion and two ends of the suture extending away from the material may be positioned in the first opening and extend into the passageway. The material may be secured relative to the body portion by securing the two ends of the suture in the passageway relative to the body portion near the second opening. For example, a rotator cuff tendon may be secured to a humerus by a suture that is placed in the tendon and has two ends that extend through a passageway in the humerus having one opening near the rotator cuff and a second opening positioned away from the rotator cuff, such as at a lateral side of the humerus. The two ends of the suture may be positioned in the passageway and secured at or near the second opening at the lateral side of the humerus. In one embodiment, a suture fixation device may be positioned near the second opening to help secure the two suture ends.

As a further example, a suture fixation device used to help secure a suture relative to a passageway in bone may be arranged so as to secure the suture relative to the bone at an opening into the bone by having a portion of the suture fixation device positioned outside of and adjacent the opening in contact with portions of cortical bone. By securing the suture (and potentially a tissue or other material engaged with the suture) relative to the bone by contact of the suture fixation device with cortical bone, the suture may be more securely fixed as compared to devices that engage with softer cancellous bone.

In another aspect of the invention, a suture fixation device may include a body having an inner end and an outer end and a pathway extending between the inner and outer ends. The inner end may be arranged to be positioned in a hole in a body portion, such as bone. The body may include a restriction in the pathway that relatively freely permits movement of a suture through the pathway in a first direction and inhibits movement of the suture through the pathway in a second direction opposite the first direction. Such a feature may help maintain tension on the suture, e.g., while a knot is being formed in the suture.

As a further example, a passageway formed through a body portion, such as bone, may be made by first and second intersecting holes. That is, the first and second holes may be formed into the body portion so each originate at different starting points and intersect within the body portion. The holes may be formed at an angle to each other or may be colinear. A guide may be used to define the location of the first and/or second hole, e.g., may be used to guide the movement of a bone perforator when forming the first and/or second holes, and/or may be used to locate a starting point for the formation of the first or second holes. In addition, or alternately, the guide may be used to pass a suture through the passageway. For example, a portion of the guide may be secured relative to one or both of the holes and a suture or other material may be passed through the guide portion when passing the suture or other material through the passageway.

In a further example, a guide apparatus may be used to form one or two holes used to form a passageway in a body portion and/or to pass a suture or other material through the passageway. For example, a first guide member may be secured relative to a first hole formed in bone. A second guide member may be arranged so that its orientation relative to the first guide member is known. The second guide member may be used to guide the identification of a starting point for the formation of the second hole and/or guide the movement of a bone perforator to form a second hole into the bone so that the orientation of the second hole is arranged in a known way relative to the first hole. Alternately, the second guide member may be secured relative to a second hole that intersects with the first hole. The first and second guide members may be positioned relative to each other, e.g., using a reference structure, so that distal ends of the guide members are positioned adjacent each other in the passageway. Suture or other material may be introduced into one of the guide members and fed to a point adjacent the other guide member. The suture or other material may be retrieved from the guide member, for example, by a hook-shaped member, grasper or other suture retriever.

In another example a passageway formed by two intersecting holes, for example, and without limitation, at a 90 degree angle, such that the passageway is generally not straight may be altered so as to form a straightened pathway. For example, the two intersecting holes may respectively originate from first and second openings at separated locations on a body portion, such as bone. A suture or suture-like material may be passed through the passageway and manipulated so as to form a more straight pathway between the first and second openings. In one embodiment, the suture or suture-like material (e.g., a wire) may be tensioned and moved so as to cut and/or crush cancellous bone between the first and second openings. This may provide a straightened pathway for suture in the passageway.

Various aspects of the invention may be used in an open surgical procedure or in a closed procedure, such as an arthroscopic procedure. For example, a guide apparatus may be used in an open or closed procedure as an aid in forming a passageway through a body portion, for passing a suture or other material through a passageway, or for both forming a passageway and passing a suture or other material through the passageway. In a closed procedure, each guide member in the apparatus may be passed through respective portals. Portions of the guide apparatus may remain outside of the body, however, such as the reference structure. Also, various aspects of the invention may be used in any suitable surgical or other procedure involving any suitable body portions, such as bone, muscle, skin, vascular structures, digestive structures or other tissue, implants, mesh, or other medical devices, etc.

In another example a needle used to place a suture in a material, such as a rotator cuff tendon and/or implant, may be engaged with a cannula, such as an arthroscopic cannula. Thus, the needle may be operated to place the suture by manipulating the cannula. The needle and cannula may have complementary locking members or otherwise arranged so that the needle may be selectively engaged with the cannula. Thus, the cannula may be usable as a standard arthroscopic cannula, and if needed, may be engaged with the needle. Thereafter, the needle and cannula may be used to place the suture in the selected tissue.

These and other aspects will be apparent from the following description.

FIG. 3 shows a schematic diagram of a surgical repair. As discussed above, although aspects of the invention are described with reference to a rotator cuff repair for ease of reference and understanding, aspects of the invention may be used in any surgical or other procedure, and may involve any suitable body portions, such as bone, muscle, other tissue or combinations thereof, vascular structures, digestive structures, medical implants or other devices, etc. Thus, aspects of the invention are in no way limited to the specific embodiments and examples described herein.

In this illustrative embodiment, a rotator cuff tendon 2 is secured by a suture 3 relative to a humerus 1. The suture 3 is placed in the tendon 2, for example, using a mattress stitch or other arrangement, and is passed through a passageway 5 formed through the humerus 1. In this embodiment, the passageway 5 is formed by first and second intersecting holes. A first hole 51 is formed vertically as shown in Fig. 3 from a first opening at or near a margin between the articulating surface 11 and the greater tuberosity 12 of the humerus 1. The second hole 52 is formed horizontally as shown in Fig. 3 from a lateral position on the humerus 1. The suture 3 is secured at the second opening of the second hole using a suture fixation device 4 that is positioned adjacent the second opening. Although in this embodiment the first and second holes 51 and 52 are arranged at approximately right angles, the first and second holes may be arranged at any suitable angle and may be colinear (i.e., at a 180 degree angle relative to each other).

A wire, other material or the suture 3 may be manipulated in the passageway 5 so as to cut through or crush the relatively soft cancellous bone of the humerus in the passageway 5 so that the suture follows a relatively straight path between the first and second openings into the first and second holes 51 and 52. The relatively straight pathway may be formed by a "flossing" operation, such as by using a wire that is passed through the passageway 5 and is manipulated, e.g., tensioned and reciprocally drawn between the first and second openings, so as to cut through or crush the cancellous bone, thereby forming a relatively straight path for the suture 3.

When deciding where to locate the first hole 51 for the passageway 5, a surgeon often will wish to first determine the final position for the tissue relative to the bone. To do so, the surgeon may wish to place a suture in the tendon 2 and tension the suture 3 (and thus the tendon 2) so that a desired position for the first hole 51 may be determined, e.g., based on the position of the tendon 2 relative to the bone when under tension.

In various aspects of the invention, a suture may be placed in the tendon or other tissue 2 using any suitable technique, such as a standard suturing needle and forceps, specialized suturing devices, and so on. However, in one aspect of the invention, use of a needle having a hook-shaped or curved end portion may be preferred. Figs. 4-6 show an embodiment of a needle 6 having a hook-shaped tissue engaging portion 61 at a distal end in accordance with the invention. In the illustrated embodiment, the tissue engaging portion 61 of the needle 6 has a semi-circular shape and is arranged at an angle, such as 90 degrees to a longitudinal axis of a straight portion 62 of the needle 6. The needle 6 may be formed as a hollow tube so that the suture 3 may pass through the needle 6. Suture may be loaded in the hollow portion of the needle 6 before the surgical procedure is begun, e.g., at the time of manufacture of the needle, or at any suitable time, such as during the surgical procedure. In some cases, the suture may be fed into the hollow portion of the needle 6 before the tissue engaging portion 61 is formed, e.g., by bending a tube to form a curved end shape.

The arrangement of the needle 6 may allow placement of a mattress stitch in the tissue 2 by rotating the needle as shown in Figs. 4-6 so that a tip of the tissue engaging portion 61 passes through a top side of the tissue 2 and exits from a bottom side of the tissue 2 as shown in Fig. 5, and then passes upwardly through the tissue 2 to reemerge at a top side of the tissue 2 as shown in Fig. 6. At this point, the suture 3 extending from the tip of the tissue engaging portion 61 may be grasped, such as by forceps or other gripping device, and the needle 6 may be rotated in reverse so as to again position the needle 6 as shown in Fig. 4, thereby leaving the suture 3 positioned in the tissue 2 to form a mattress stitch. During the passage of the suture, the tissue or other material may be held in place, or may be manipulated, by a grasper or other device inserted into the lumen of the cannula. The tissue or other material may also be held in place, or manipulated, by another device, such as a grasper or clamp positioned external to the cannula.

The tissue engaging portion 61 of the needle 6 may have any suitable shape and may be arranged in a plane that is transverse at any angle to an axis of rotation of the tissue engaging portion 61 when placing a suture in tissue. That is, although in the illustrated embodiment the tissue engaging portion 61 has a semi-circular form that lies in a plane at 90 degrees to the rotation axis of the tissue engaging portion 61 when placing a suture, the tissue engaging portion 61 need not have a semi-circular form and may lie at any desired angle to the rotation axis. For example, the tissue engaging portion 61 may be arranged so as to place an inclined mattress stitch in a tissue 2. Further, the needle 6 need not be used only to form a mattress stitch, but rather may be used to form any other suitable stitch type. Also, it is not necessary that the tissue engaging portion 61 of the needle 6 lie in a single plane. Instead, the tissue engaging portion 61 may not lie in a single plane, e.g., may have a corkscrew-type or partially helical configuration.

In one aspect of the invention, all or portions of a tissue repair procedure may be performed arthroscopically. In this case, and as is known in the art, one or more cannulas may be provided in one or more portals formed in the patient so as to provide access to the operative site. In one aspect of the invention, a needle used to place a suture in a tissue, such as the needle 6 shown in Fig. 4, may be used in an arthroscopic procedure. For example, the needle 6 may be secured to a cannula so that the needle may be operated by manipulation of the cannula.

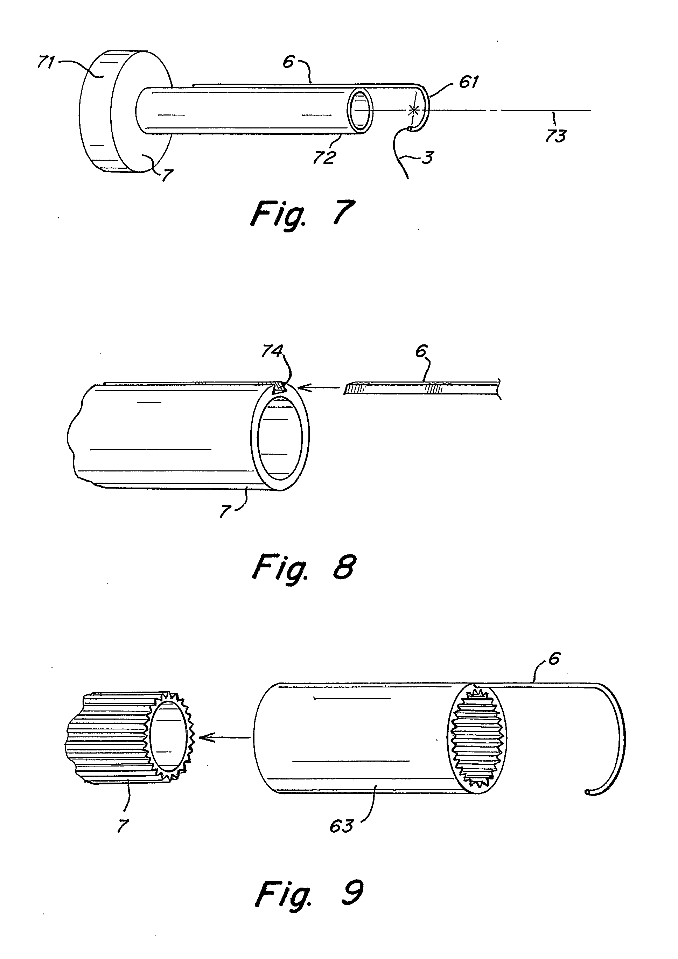

Fig. 7 shows an illustrative embodiment of a needle 6 that is secured to a cannula 7. The cannula 7 may have any suitable features found in cannulas used for closed or minimally-invasive surgical techniques, such as one or more valves to resist fluid flow through the cannula 7, an opening through which to introduce a fluid pressure or vacuum, spiral threads or other features on the cannula to aid in placement of the cannula in a portal and/or to help prevent inadvertent removal of the cannula from the portal, and so on. The cannula 7 may be arranged for any type of procedure, such as arthroscopic procedures.

The needle 6 may be secured to the cannula 7 in any suitable way. For example, the needle 6 may be molded into the body of the cannula 7, inserted into the wall of the cannula, may be secured by adhesive, welding, clamps, fasteners, interlocking channels, open channels, or any other suitable device. A proximal end of the needle 6 may terminate at any suitable point, such as midway between a proximal end 71 and a distal end 72 of the cannula 7 as shown, or, more preferably at a position proximal to the proximal end 71. By having the proximal end of the needle 6 positioned proximally of the cannula 7, a user may be better able to access the suture 3 entering the proximal end of the needle 6. The needle 6 may also be axially movable relative to the cannula, e.g., so that the tissue engaging portion 61 may be moved axially so as to extend away from or toward the distal end 72 of the cannula 7. In addition, although the needle 6 is shown as positioned on an outer surface of the cannula 7, the needle 6, or at least a portion thereof, may be molded into the cannula 7, positioned within the cannula lumen, positioned within the cannula wall, may be arranged within a groove on the outer surface of the cannula, and so on. Although the needle 6 is shown as arranged in an approximately straight fashion along the length of the cannula 7, the needle 6 may be bent, curved or arranged in any suitable way, such as following a spiral path around an outer surface of the cannula 7.

In one illustrative embodiment, a semicircular-shaped tissue engaging portion 61 of the needle 6 may be arranged relative to the cannula 7 so that a centerpoint of the semicircle lies on a central longitudinal axis 73 of the cannula lumen. Thus, when the cannula 7 is rotated about the central longitudinal axis 73, the tissue engaging portion 61 may travel in a circular path about the axis 73. However, it should be understood that the tissue engaging portion 61 may be arranged in any suitable way relative to the axis 73. Further, a plane in which the tissue engaging portion 61 lies (if present) may be arranged at any angle transverse to the axis 73, and thus need not be arranged at an angle of 90 degrees to the axis 73, as shown in Fig. 7.

In one aspect of the invention, the needle 6 may be removeably engaged with the cannula 7 so that the needle 6 can be selectively engaged or disengaged with the cannula 7. For example, a cannula 7 may be positioned in a portal in use during a surgical procedure without an attached needle 6. At some point during the procedure, the surgeon may wish to attach a needle 6 to the cannula 7 and manipulate the cannula 7 so as to use the needle 6 to place a suture in a tissue. The needle 6 may be secured to the cannula while the cannula remains in place in the portal (e.g., by inserting the needle 6 into the cannula lumen), or the cannula may be removed from the portal, the needle attached, and the cannula and attached needle inserted into the portal.

Fig. 8 shows one illustrative embodiment in which a needle 6 may be removably secured to a cannula 7. In this embodiment, the cannula 7 includes a dovetail-shaped groove 74 into which a correspondingly shaped portion of the needle 6 is inserted. The complementary locking arrangement used by the cannula 7 and the needle 6 need not necessarily be dovetail-shaped as shown in Fig. 8, but rather may have any suitable arrangement. For example, the cannula 7 may have an oval-shaped channel that receives an oval-shaped portion of the needle 6. Thus, the needle 6 may be selectively secured to the cannula 7 so that rotation or other manipulation of the cannula 7 can cause the needle to be manipulated so as to place a suture in a tissue. The complementary locking arrangement between the needle 6 and the cannula 7 may also allow for axial movement of the needle 6 relative to the cannula 7, e.g., so the tissue engaging portion 61 can be moved relative to the distal end 72 of the cannula 7.

Fig. 9 shows an alternative embodiment in which a needle 6 is fixed to a sleeve member 63 that has one or more complementary locking features that mesh with or otherwise engage with complementary features on the cannula 7. In this embodiment, the complementary locking features have a tooth-like or gear-like form, but the complementary locking features may be arranged in any suitable way. Accordingly, in this embodiment, the needle 6 may be secured to the cannula 7 by sliding the sleeve 63 over the distal end 72 of the cannula 7. It will be understood that rather than having a sleeve 63 that fits over the cannula 7, the sleeve 63 may fit within the internal lumen of the cannula 7, or within a slot in the cannula 7, if desired.

Once a suture is placed in the tissue, such as a rotator cuff tendon, the tissue may be tensioned to determine a location for the opening of the first hole 51 to be formed in the bone. When performing a rotator cuff repair, a first hole 51 of the passageway 5 may be formed vertically from a superolateral position so that the first hole 51 is generally aligned along the length of the humerus 1 and extends into the bone from an opening formed at the margin between the articulating surface 11 and the greater tuberosity 12. This first hole 51 may be formed using a perforator, such as a drill, awl, punch or other suitable device. As with other procedures performed, the first hole 51 may be formed using an arthroscopic portal at a superolateral position, or may be formed in an open surgical procedure.

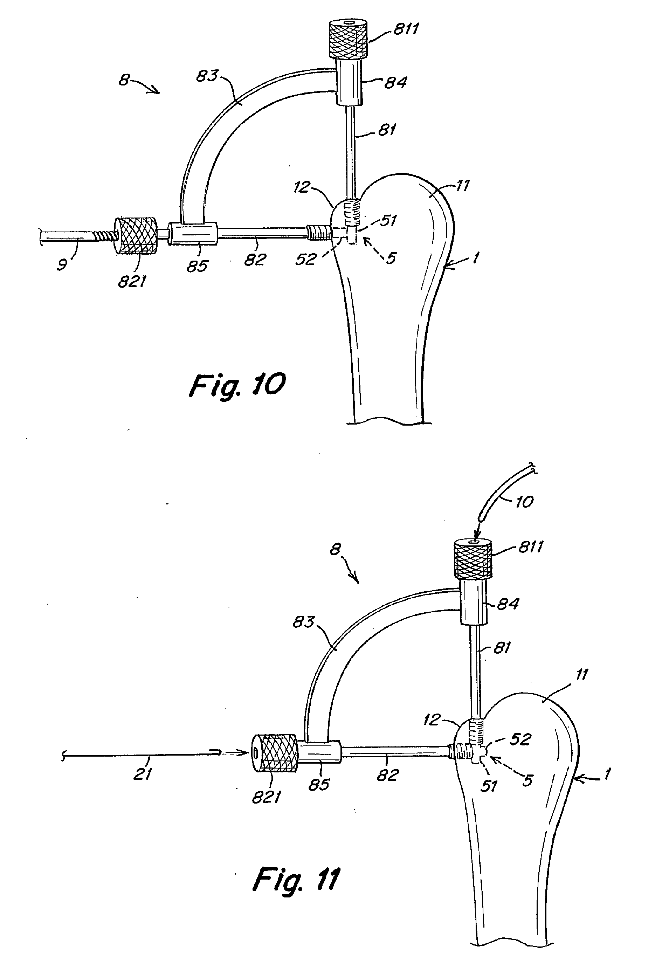

As an example, a guide apparatus may be used to form the first and/or second holes of the passageway (e.g., used to locate a starting point or opening for the first and second holes, used to orient a bone perforator when making the holes, or used alone to form the first and/or second holes), or may be used to help feed a suture or suture-like material through the passageway. For example, a first guide member 81 may be secured relative to the first hole 51, as shown in Fig. 10. The first guide member 81 may be part of a guide apparatus 8 used to guide the formation of holes used to form a passageway in bone and/or to pass a suture or other material through the passageway. In the illustrated embodiment, the first hole 51 has been formed in a vertical direction along the length of the humerus 1, e.g., by drilling the hole in a freehand manner. (Alternately, the first hole 51 may be formed by forcing the first guide member 81 into the bone as with an awl or similar instrument.) The first guide member 81 may include a feature to help secure the first guide member 81 relative to the first hole 51, such as a threaded distal end that allows the first guide member 81 to be screwed into the bone to a desired depth in the first hole 51. It should be understood, however, that the distal end of the first guide member 81 need not be threaded, but instead may unthreaded and inserted into the first hole 51. Alternately, the distal end of the first guide member 81 may be positioned outside of, but adjacent to, the first hole 51 so that a lumen in the first guide member 81 aligns with the first hole 51. The first hole 51 may be formed so as to be deeper than thought to be needed, e.g., 0.5 cm deeper than a hole depth believed to be required. This overdrilling of the first hole 51 may allow for more flexibility in positioning the first guide member 81 to a desired depth in the bone.

The first guide member 81 may be arranged with respect to a reference structure 83 used to position first and second guide members 81 and 82 relative to each other with respect to the passageway 5, as is discussed in more detail below. In this illustrative embodiment, the reference structure 83 is arranged so that the first and second guide members 81 and 82 are positioned at a 90 degree angle relative to each other when engaged with the reference structure 83. However, the reference structure 83 may be arranged in any suitable way so as to orient the first and second guide members 81 and 82 at any desired angle relative to each other, including arranging the first and second guide members 81 and 82 in a co-linear fashion. Further, the reference structure 83 may be made so as to be adjustable, thereby allowing the orientation of the first and second guide members 81 and 82 to be changed. For example, the arc-shaped connecting portion of the reference structure 83 may be made so as to be adjustable in length, e.g., having one arc-shaped portion sliding relative to another arc-shaped portion to allow adjustment of the length of the connecting portion. Alternately, or in addition, engagement portions 84 and 85 of the reference structure 83 that engage with the first and second guide members 81 and 82 may be adjustable in orientation relative to the arc-shaped connecting portion. In short, the reference structure 83 may be arranged in any suitable way so as to allow adjustment in the orientation of the guide members 81 and 82.

In this illustrative embodiment, the engagement portions 84 and 85 include sleeves that receive at least a portion of the guide members 81 and 82, e.g., the guide members 81 and 82 may be received in bores in the sleeves. The sleeves may be arranged so that the guide members 81 and 82 are movable linearly along their longitudinal axes and rotationally about their longitudinal axes relative to the engagement portions 84 and 85, but otherwise may be relatively restricted in their range of movement. When a stop on the first guide member 81, such as a knob 811 on the proximal end of the guide member 81, contacts an engagement surface on the reference structure, such as a portion of the engagement portion 84, the second guide member 82 may be positioned by the reference structure 83 so that its longitudinal axis passes a point adjacent the extreme distal end of the first guide member 81. Thus, the second guide member 82 may be used to guide the use of a perforator 9 (such as a drill, punch, awl or other bone perforating device) so that the perforator 9 forms a second hole 52 that intersects with the first hole 51 at a location adjacent the distal end of the first guide member 81. As discussed above, the guide member 82 may guide the movement of the perforator 9, e.g., guide the movement of a drill or punch inserted into a lumen of the guide member 82 as shown, or may guide a starting location for forming the second hole, e.g., be used to mark or otherwise determine a starting location for the perforator 9, but otherwise not interact with the perforator 9. By adjusting the depth of the first guide member 81 in the first hole 51, the location where the second hole 52 is formed can be adjusted in position (e.g., in a vertical direction as shown in the figures). For example, by screwing the first guide member 81 into or out of the first hole 51, a surgeon may select a location where the second hole 52 is to be formed in the bone.

In another embodiment, the engagement portion 85 may itself function as a perforator guide with the second guide member 82 being withdrawn from the engagement portion 85. Although in this illustrative embodiment the engagement portions 84 and 85 are shown as relatively short cylindrical sleeves, the engagement portions 84 and 85 may be arranged in any suitable way, e.g., may be elongated so as to more closely approach the humerus 1 and provide improved guidance for a perforator 9 and/or the first and second guide members 81 and 82. Further, the first guide member 81 may be arranged so that is rotationally movable about its longitudinal axis relative to the reference structure 83, but is otherwise held by the engagement portion 84 so that the first guide member 81 is not movable axially. This may aid is appropriately positioning the first guide member 81 and reference structure 83 when forming the second hole 52.

Upon formation of the second hole 52, the second guide member 82 may be screwed into the second hole 52 until a stop on the second guide member 82, such as a knob 821 at a proximal end of the guide member 82, contacts an engagement surface on the engagement portion 85, such as a portion of the sleeve. In this configuration shown in Fig. 11 (stops on the first and second guide members 81 and 82 engaged with respective engagement surfaces on the reference structure 83), the extreme distal ends of the first and second guide members 81 and 82 may be adjacent to each other in the passageway 5 formed by the first and second holes 51 and 52. Accordingly, a surgeon may be assured that if the first and second guide members 81 and 82 are positioned within the bone and stops on the guide members 81 and 82 are respectively in contact with appropriate engagement surfaces on the guide apparatus 8, the extreme distal ends of the guide members 81 and 82 will be positioned adjacent each other. Thus, the surgeon may be assured that a wire 10 or other element may be fed into one of the guide members and retrieved from the other of the guide members, e.g., using a retriever 21 having a hook at a distal end. Such an arrangement may be advantageous when using the guide apparatus 8 in an arthroscopic procedure where the operative site may not be easily visualized.

Although in the above embodiment, stops on the first and second guide members 81 and 82 contact corresponding engagement surfaces on the engagement portions 84 and 85, the guide members 81 and 82 may be positioned relative to the reference structure 83 in any suitable way. For example, the guide members 81 and 82 may have indicator marks on them that may be aligned with a portion of the engagement portions 84 and 85, respectively. The alignment of certain indicator marks on the guide members 81 and 82 may be used to indicate, for example, that the distal ends of the guide members 81 and 82 are adjacent each other. Those of skill in the art will understand that the position of the guide members 81 and 82 relative to the reference structure 83 and relative to each other may be determined in other ways. For example, the first and second guide members 81 and 82 need not necessarily be positioned so that their distal ends are adjacent in the passageway to assist in feeding a suture from one guide member to the other. Instead, the first and second guide members 81 and 82 may be made to suitably communicate with the passageway in any way so as to facilitate feeding of the suture.

In this illustrative embodiment, the first guide member 81 is shown as having a smaller diameter (at least at the distal end) than the second guide member 82. This may allow the guide apparatus 8 to be used with an arrangement where the first hole 51 is smaller than the second hole 52. A relatively small first hole 51 may allow for more rapid healing and/or provide additional space for other holes in the margin, if needed. However, it should be understood that the guide apparatus 8 and/or the holes that form the passageway 5 may be made in any suitable way, e.g., the first and second holes 51 and 52 may have the same diameter or the first hole 51 may have a larger diameter than the second hole 52.

The guide apparatus 8 may also include additional guide members if desired, e.g., to provide for the formation of a third hole that is formed in the margin and is approximately parallel to the first hole 51, but also intersects with the second hole 52. Similarly, the guide apparatus 8 may include two pairs of guide members like that in the illustrative embodiment that are arranged to form side-by-side passageways 5 in the bone or other body portion.

Although in this illustrative embodiment, the guide apparatus 8 is used to guide the formation of the second hole 52, the guide apparatus 8 need not necessarily be used to guide the formation of the second hole 52. That is, the guide apparatus 8 may be used only to help feed the wire 10, suture or other material through a passageway that is preformed in the bone or other body portion. In addition, the first and second guide members 81 and 82 may be arranged so that the members 81 and 82 can be secured in a body portion without requiring holes to be predrilled or otherwise formed. Thus, in one embodiment, the first and second guide members 81 and 82 may be arranged like an awl or other device capable of forming a hole in a body portion, e.g., capable of being forced into bone, forming the passageway 5 by their entry and/or providing a means to help feed a wire, suture or other material through the passageway 5.

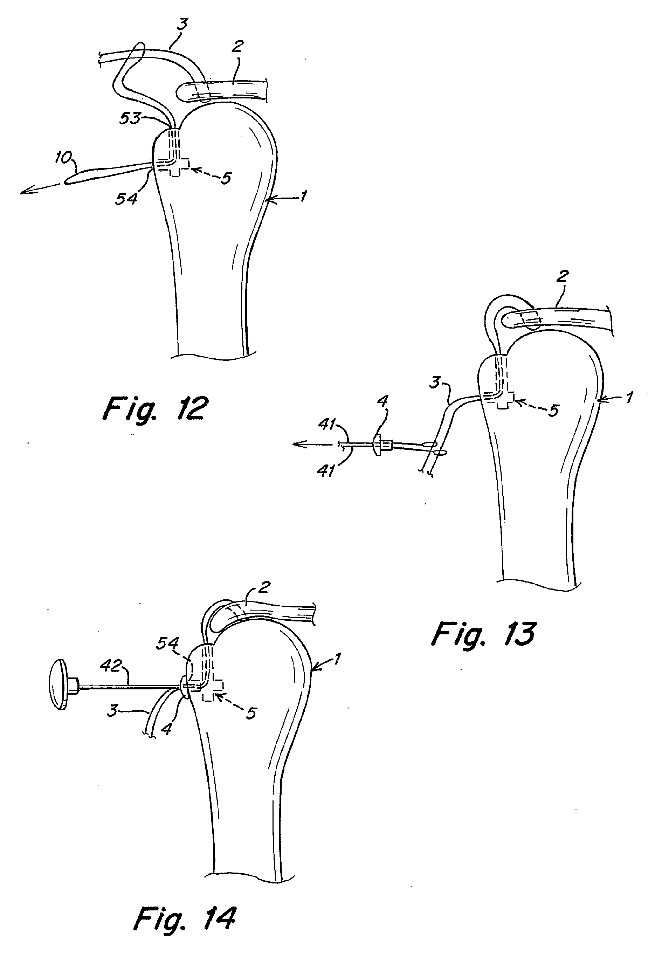

Once the wire 10, suture or other material has been passed through the passageway 5, as shown in Fig. 12, the wire 10 may be used to pull the suture 3 through the passageway 5. Prior to being used to pull the suture 3 through the passageway 5, the wire 10 or other material may be used to create a relatively straight pathway for the suture 3 once the suture 3 is tensioned and fixed in place. For example, the wire 10 may be tensioned between the first and second openings 53 and 54 of the first and second holes 51 and 52 or otherwise manipulated so as to cut or crush the body portion, e.g., bone, between the first and second openings 53 and 54. Such manipulation of the wire 10 may perform a kind of "flossing" effect in the bone, allowing the suture 3 to follow a more straight pathway through the passageway 5, reducing the length of suture 3 needed between the rotator cuff 2 and a point of fixation of the suture 3, e.g., near the second opening 54. The wire 10 may have barbs or other saw-like features to aid in cutting bone and forming the pathway. Of course, the more straight pathway could be formed by manipulating the suture 3 itself, e.g., by tensioning the suture 3 when securing the tendon or other material. Reducing the length of suture 3 in the passageway 5 may improve the suture's ability to maintain appropriate tension on the rotator cuff 2, e.g., by reducing the amount of stretch of the suture when under tension.

After the suture 3 has been passed through the passageway 5, the suture 3 may be engaged with a suture fixation device 4 as shown in Fig. 13. Although use of a suture fixation device is not required, the suture fixation device 4 may improve an ability to securely fixate the suture 3 (and the tissue 2) relative to the bone. The suture fixation device 4 may be arranged in any suitable way, but in this illustrative embodiment has an arrangement similar to a button. For example, the suture fixation device 4 may have two through holes formed in a disk-shaped member through which leading ends of the suture 3 are passed. The suture ends 3 may be passed through respective holes in the suture fixation device 4 using one or more feed members 41. The feed members 41 may have an elongated shape that is passed through a respective hole in the suture fixation device 4. A loop at one end of the feed member 41 may receive an end of the suture 3 and thereafter the feed member 41 may be pulled through a respective hole in the suture fixation device 4 so as to pull the suture 3 through the hole. The feed member 41 may include one or more flat plates or strips, e.g., made of metal or plastic, with a hole or recess to accept suture. The flat configuration may allow for easy passage through the restriction portion of the device. Although some resistance may be encountered when feeding the portion of the feed member 41 that engages the suture through the restriction, permanent damage or other compromise of the resistive properties of the restriction may be avoided. Of course, it should be understood that the suture 3 may be fed through the suture fixation device 4 in any other suitable way. When performing this technique arthroscopically, the suture 3 may be fed through the suture fixation device 4 either inside or outside of the body cavity.

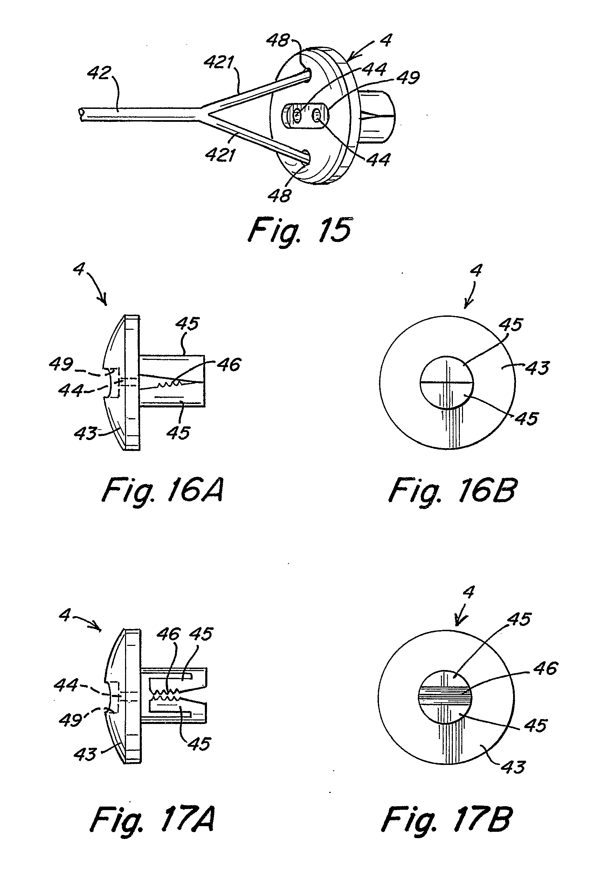

As shown in Fig. 14, after the suture 3 is engaged with the suture fixation device 4, the suture fixation device 4 may be positioned relative to the second opening 54 of the passageway 5 using an applier 42 which removably engages with the suture fixation device 4 and may be selectively disengaged from the suture fixation device when the suture fixation device 4 is positioned as desired. As shown in Fig. 15, the applier 42 may have a pair of tines 421 that engage with recesses or other features on the suture fixation device 4 so as to removably engage with the suture fixation device 4. The tines 421 may be resilient so that the tines are squeezed together when engaged with the suture fixation device 4. Thus, an elastic force biasing the tine ends apart may help maintain engagement of the tines with the grooves 48 in the suture fixation device 4. Alternately, the tine ends may be force-fit into grooves 48 in the suture fixation device so that engagement is maintained based on friction. Of course, it will be understood that the applier 42 may engage with the suture fixation device 4 in any other suitable way, such as a screw-in or snap configurations.

With the suture fixation device 4 in place relative to the second opening 54, the suture 3 may be tensioned so as to appropriately position the rotator cuff 2 relative to the humerus 1. At this point, the suture 3 may be fixed relative to the suture fixation device 4, such as by tying a knot with the suture ends. Thus, the suture fixation device 4 may provide not only a structure to support the suture knot, but also may spread the force of the suture 3 to portions of the relatively hard cortical bone surrounding or otherwise adjacent to the second opening 54. By having the suture fixation device 4 engage with this cortical bone, the suture fixation device 4 may provide a relatively stable and secure fixation point for the suture 3. The suture fixation device 4 may also incorporate a mechanism for knotless fixation of the suture, such as an interference pin, a locking passageway, a locking cap, etc.

Although in the illustrative embodiment described above both ends of the suture 3 are passed through the passageway 5 and secured at or near the second opening 54 of the passageway 5, the suture 3 may be secured in other ways, such as by passing one end of the suture 3 through the passageway 5 and passing another end of the suture 3 around the outside of the bone (e.g., over a portion of the greater tuberosity) where it is secured to the other suture end. In another embodiment, two passageways 5 may be formed through the bone and one end of the suture 3 may be passed through one passageway and the other end of the suture 3 may be passed through the other passageway. The suture ends may then be secured to each other at or near respective second openings of the passageways 5 on the lateral side of the humerus 1. In yet another embodiment, two or more first holes 51 may be formed so as to intersect with one or more second holes 52. Suture 3 may be passed through the two or more first holes 51 and be secured at the second opening 54 of the one or more second holes 52. Such an arrangement may allow for the use of a single second hole 52 and suture fixation device 4 to secure the rotator cuff at two or more points on the humeral head using two or more sutures that pass through different first holes 51. Other suture fixation techniques may be used as desired.

Figs. 16A-B and 17A-B show illustrative embodiments of suture fixation devices 4 in accordance with the invention. In these embodiments, the suture fixation device 4 includes a restriction in a pathway through the suture fixation device 4 so that suture or other material passing through the suture fixation device is relatively freely moved in one direction through the pathway, but movement of the suture or other material in the other direction in the pathway is resisted. For example, movement of a suture though the suture fixation devices shown in Figs. 16A and 17A in a direction to the left in the side view of the suture fixation devices 4 may be freely allowed, while movement of the suture toward the right may be resisted. This may aid in tensioning the suture 3 because the suture 3 may be pulled from the second hole 52 through the suture fixation device 4 until the rotator cuff or other tissue is appropriately positioned. Thereafter, tension on the suture may be temporarily released, e.g., in preparation for forming a knot, but movement of the suture back through the suture fixation device 4 may be resisted so that the rotator cuff or other tissue is maintained in place until the suture knot is tied or otherwise is secured.

In the Fig. 16 embodiment, the suture fixation device includes an outer end having a flange portion 43 that is sized and arranged to contact the cortical bone adjacent the opening in the passageway at which the suture fixation device 4 is positioned, e.g., the second opening 54. One or more pathways 44 may be formed through the suture fixation device 4, such as by a hole or holes formed through the flange 43 (see also Fig. 15). Instead of having multiple holes, the pathway 44 may include a single slot arranged to receive one or more sutures. A recess 49 may be provided in the flange portion 43 to receive one or more knots, if formed with the suture(s) in the pathway 44. A pair of duck bill members 45 at an inner end of the suture fixation device 4 may extend rearwardly from the flange 43 and may be arranged as to be positionable in the second hole 54. The duck bill members 45 may be separated from each other by a groove that extends across the inner end of the suture fixation device 4 and be resiliently biased toward each other so as to resist the passage of suture or other material through the pathway 44. One or both of the duck bill structures 45 may include serrations 46 or other features that may aid in engaging a suture or other material. In the Fig. 16 embodiment, the groove separating the duck bill structures 45 extends to the flange portion 43 so that the structures 45 are pivotable at a point near the flange portion 43.

The Fig. 17 embodiment similarly includes a flange 43 and one or more pathways 44. Duck bill structures 45 are also provided. However, in this embodiment rather than being hinged at a point near respective connection points with the flange 43, the duck bill portions 45 are hinged at a point positioned away from the flange 43. Providing the effective hinge points for the duck bill structures 45 in this manner may provide improved engagement of the duck bill structures 45 with a suture or other material when the suture is urged to move from the outer end toward the inner end through the pathway 44. That is, if the suture is pulled to move toward the inner end, serrations 46 or other features will engage with the suture and increased force on the suture will cause an increased force urging the duck bill structures 45 to move toward each other and further squeeze the suture. The duck bill structures in the Fig. 16 and 17 embodiments or other suitable suture engagement arrangements (such as interference pins, locking caps, locking internal hubs, etc.) may provide a knotless fixation for the suture. Alternatively, the structures may resist movement of the suture so as to aid the surgeon's ability to maintain tension on the suture while forming a knot.

Although this embodiment depicts the flange of the device resting on the outer cortical surface of the bone, the device may be positioned in a hole which has a counterbore, or countersink, in order to prevent any interference between the flange and other bone or tissues that may come in contact with the site either at rest or during movement. Even in the case where the device is positioned in a counterbore or countersink feature, the device may contact cortical bone. Alternately, the device may only contact the outer, cortical surface of the bone, and not extend into a hole in the bone.

Of course, it should be understood that suture fixation devices may be provided in any suitable form. For example, the duck bill portions 45 extending from the flange 43 in the Figs. 16 and 17 embodiments may be sized to closely fit into a mating hole formed in bone. This close fit may help in maintaining the suture fixation devices 4 in a desired position in the bone. Alternately, the duck bill structures 45 may be formed so as to be tapered on their outer surfaces. Thus, when the suture fixation device 4 is inserted into a hole in the bone, the tapered surfaces of the duck bill structures 45 may contact the sides of the hole and urge the duck bill structures to move toward each other as the suture fixation device 4 is pressed into the hole. In another embodiment, a portion of the suture fixation device 4 that is inserted into a hole may be threaded or otherwise be arranged so as to engage the hole and help prevent the suture fixation device from falling from the hole, e.g., before the suture is secured in place. There are many variations of the mechanism form to retain the suture with respect to the device. Some of these forms may require a knot for final fixation. Other capturing mechanisms may provide sufficient locking of the suture such that a knot is not required. Theses are typically known as "knotless" devices.

Having thus described several aspects of at least one embodiment of this invention, it is to be appreciated various alterations, modifications, and improvements will readily occur to those skilled in the art. Accordingly, the foregoing description and drawings are by way of example only.

## Claims

1. An apparatus for use with a passageway in a surgical procedure, comprising:
a reference structure (83);
a first guide member (81) connected to the reference structure and adapted to be arranged in a first position relative to the reference structure (83), the first guide member (81) having distal and proximal ends and a first passage leading to the distal end, the distal end of the first guide member (81) being arranged to be inserted in a first hole in a bone so as to position the first guide member (81) and the reference structure (83) relative to the bone;
a second guide member (82) connected to the reference structure (83) and adapted to be arranged in a second position relative to the reference structure (83), the second guide member (82) having distal and proximal ends and a second passage leading to the distal end, the second passage being arranged, with the first guide member (81) inserted in the first hole, to guide a bone perforator in formation of a second hole into bone that is at an angle to and intersects with the first hole; **characterised by**
a retriever (21) arranged to retrieve, from the passage of the first or second guide member (81,82), a wire, suture or other element fed into the passage of the other of the first or second guide member (81,82).

2. The apparatus of claim 1, wherein:
the first and second guide members (81,82) each include a stop (811, 821); and
the reference structure (83) is constructed and arranged to engage with the stops (811,821) on the first and second guide members (81,82) so that when the stops (811,821) of the first and second guide members (81,82) are engaged with the reference structure (83), the distal ends of the first and second guide members (81,82) are adjacent each other.

3. The apparatus of claim 1 or 2, wherein the distal ends of the first and second guide members (81,82) are adapted to be forcibly inserted into bone.

4. The apparatus of any one of claims 1 to 3, wherein the first and second passages in the first and second guide members (81,82) extend from proximal ends of the first and second guide members (81,82) to the distal end of the first and second guide members (81,82).

5. The apparatus of any one of claims 1 to 4, wherein the first passage has a cross sectional size that is smaller than a cross sectional size of the second passage.

6. The apparatus of any one of claims 1 to 5, further comprising at least one bone perforator (9) constructed and arranged to form a hole in bone while being guided in orientation by the passage of the second guide member (82).

7. The apparatus of any one of claims 1 to 6, wherein the retriever (21) is arranged to retrieve a suture from the passage of the first or second guide member (81,82) and the apparatus further comprises a suture fixation device constructed and arranged to be positioned relative to a passageway in bone and aid in securing a suture at least partially positioned in the passageway.

8. The apparatus of any one of claims 1 to 7, wherein the reference structure (83) and the first and second guide members (81,82) are constructed and arranged to be used in an arthroscopic procedure to form a passageway through bone.

9. The apparatus of any one of claims 1 to 8, wherein the reference structure (83) includes a connecting member and first and second sleeves (84, 85) that respectively include first and second openings and are attached to the connecting member.

10. The apparatus of claim 9, wherein:
the first and second sleeves each include a bore and an engagement surface; and
the first and second guide members (81,82) each include a stop (811,821);
wherein the first and second guide members (81,82) are receivable in the bore of the first and second sleeves, respectively, so that the stops (811,821) engage a respective engagement surface and position the distal ends adjacent each other.

11. The apparatus of claim 10, wherein the first and second guide members (81,82) are arranged at an angle to each other when received in their respective sleeves.

12. The apparatus of claim 11, wherein the angle is approximately 90 degrees.

13. The apparatus of any one of claims 1 to 12, wherein the first and second guide members (81,82) each include a tube constructed and arranged to be positioned in an arthroscopic cannula.

14. The apparatus of any one of claims 1 to 13, wherein the apparatus is arranged to be used with a passageway in a humerus for performing a rotator cuff repair.

15. The apparatus of any one of claims 1 to 16, wherein the first and second guide members (81,82) and the reference structure (83) are constructed and arranged to cooperate with a passageway in a humerus with the first guide member (81) passing through a superolateral arthroscopic portal and the second guide member (82) passing through a lateral arthroscopic portal while the reference structure (83) is positioned outside of the body.

## Patentansprüche

1. Vorrichtung zur Verwendung mit einem Durchgangsweg bei einem chirurgischen Eingriff, die Folgendes umfasst:
eine Bezugsstruktur (83);
ein erstes Führungselement (81), das mit der Bezugsstruktur verbunden ist und dazu angepasst ist, in einer ersten Lage relativ zur Bezugsstruktur (83) angeordnet zu werden, wobei das erste Führungselement (81) ein distales und ein proximales Ende und einen zu dem distalen Ende führenden ersten Durchgang aufweist, wobei das distale Ende des ersten Führungselements (81) dazu angeordnet ist, in ein erstes Loch in einem Knochen eingesteckt zu werden, um das erste Führungselement (81) und die Bezugsstruktur (83) relativ zu dem Knochen zu positionieren;
ein zweites Führungselement (82), das mit der Bezugsstruktur (83) verbunden ist und dazu angepasst ist, in einer zweiten Lage relativ zu der Bezugsstruktur (83) angeordnet zu werden, wobei das zweite Führungselement (82) ein distales und ein proximales Ende und einen zu dem distalen Ende führenden zweiten Durchgang aufweist, wobei der zweite Durchgang dazu angeordnet ist, wenn das erste Führungselement (81) in das erste Loch eingesteckt ist, einen Knochenbohrer beim Bilden eines zweiten Lochs in Knochen zu führen, das unter einem Winkel zum ersten Loch verläuft und dieses schneidet, **gekennzeichnet durch:**
einen Rückholer (21), der dazu angeordnet ist, aus dem Durchgang des ersten oder des zweiten Führungselements (81,82), einen Draht, einen Faden oder ein anderes Element, der bzw. das in den Durchgang des anderen von dem ersten oder zweiten Führungselements (81,82) eingeführt wurde, zurückzuholen.

2. Vorrichtung nach Anspruch 1, wobei:
das erste und das zweite Führungselement (81,82) jeweils einen Anschlag (811, 821) umfassen; und
die Bezugsstruktur (83) dazu ausgebildet und angeordnet ist, mit den Anschlägen (811,821) an dem ersten und dem zweiten Führungselement (81,82) in Eingriff zu treten, sodass, wenn sich die Anschläge (811,821) des ersten und des zweiten Führungselements (81,82) mit der Bezugsstruktur (83) in Eingriff befinden, die distalen Enden des ersten und des zweiten Führungselements (81,82) einander benachbart sind.

3. Vorrichtung nach Anspruch 1 oder 2, wobei die distalen Enden des ersten und des zweiten Führungselements (81,82) dazu angepasst sind, gewaltsam in den Knochen eingesteckt zu werden.

4. Vorrichtung nach einem der Ansprüche 1 bis 3, wobei sich der erste und der zweite Durchgang in dem ersten und dem zweiten Führungselement (81,82) von proximalen Enden des ersten und des zweiten Führungselements (81,82) zum distalen Ende des ersten und des zweiten Führungselements (81,82) erstrecken.

5. Vorrichtung nach einem der Ansprüche 1 bis 4, wobei der erste Durchgang eine Querschnittsgröße aufweist, die kleiner ist als eine Querschnittsgröße des zweiten Durchgangs.

6. Vorrichtung nach einem der Ansprüche 1 bis 5, weiter umfassend mindestens einen Knochenbohrer (9), der dazu ausgebildet und angeordnet ist, ein Loch in Knochen zu bilden, während er in der Orientierung von dem Durchgang des zweiten Führungselements (82) geführt wird.

7. Vorrichtung nach einem der Ansprüche 1 bis 6, wobei der Rückholer (21) dazu angeordnet ist, einen Faden aus dem Durchgang des ersten oder des zweiten Führungselements (81,82) zurückzuholen und die Vorrichtung weiter eine Fadenfixierungsvorrichtung umfasst, die dazu ausgebildet und angeordnet ist, relativ zu einem Durchgangsweg in Knochen positioniert zu werden und beim Befestigen eines mindestens teilweise in dem Durchgangsweg positionierten Fadens zu helfen.

8. Vorrichtung nach einem der Ansprüche 1 bis 7, wobei die Bezugsstruktur (83) und das erste und das zweite Führungselement (81,82) dazu ausgebildet und angeordnet sind, bei einem arthroskopischen Eingriff verwendet zu werden, um einen Durchgangsweg durch Knochen zu bilden.

9. Vorrichtung nach einem der Ansprüche 1 bis 8, wobei die Bezugsstruktur (83) ein Verbindungselement umfasst und eine erste und eine zweite Hülse (84,85) umfasst, die jeweils eine erste bzw. eine zweite Öffnung umfassen und an dem Verbindungselement angebracht sind.

10. Vorrichtung nach Anspruch 9, wobei:
die erste und die zweite Hülse jeweils eine Bohrung und eine Eingriffsfläche umfassen; und
das erste und das zweite Führungselement (81,82) jeweils einen Anschlag (811,821) umfassen;
wobei das erste und das zweite Führungselement (81,82) jeweils in der Bohrung der ersten bzw. der zweiten Hülse aufgenommen werden können, sodass die Anschläge (811,821) mit einer jeweiligen Eingriffsfläche in Eingriff treten und die distalen Enden benachbart einander positionieren.

11. Vorrichtung nach Anspruch 10, wobei das erste und das zweite Führungselement (81,82) unter einem Winkel zueinander angeordnet sind, wenn sie in ihrer jeweiligen Hülse aufgenommen sind.

12. Vorrichtung nach Anspruch 11, wobei der Winkel ungefähr 90 Grad beträgt.

13. Vorrichtung nach einem der Ansprüche 1 bis 12, wobei das erste und das zweite Führungselement (81,82) jeweils eine Röhre umfassen, die dazu ausgebildet und angeordnet ist, in einer Arthroskopiekanüle positioniert zu werden.

14. Vorrichtung nach einem der Ansprüche 1 bis 13, wobei die Vorrichtung dazu angeordnet ist, mit einem Durchgangsweg in einem Humerus verwendet zu werden, um eine Rotatorenmanschettenreparatur auszuführen.

15. Vorrichtung nach einem der Ansprüche 1 bis 16, wobei das erste und das zweite Führungselement (81,82) und die Bezugsstruktur (83) dazu ausgebildet und angeordnet sind, mit einem Durchgangsweg in einem Humerus zusammenzuwirken, wenn das erste Führungselement (81) durch ein superolaterales Arthroskopieportal verläuft und das zweite Führungselement (82) durch ein laterales Arthroskopieportal verläuft, während die Bezugsstruktur (83) außerhalb des Körpers positioniert ist.

## Revendications

1. Appareil destiné à être utilisé avec une voie de passage lors d'une intervention chirurgicale, comprenant :
une structure de référence (83) ;
un premier élément de guidage (81) relié à la structure de référence et adapté pour être disposé dans une première position par rapport à la structure de référence (83), le premier élément de guidage (81) possédant une extrémité distale et une extrémité proximale et un premier passage conduisant à l'extrémité distale, l'extrémité distale du premier élément de guidage (81) étant disposée de façon à être introduite dans un premier trou percé dans un os afin de positionner le premier élément de guidage (81) et la structure de référence (83) par rapport à l'os ;
un deuxième élément de guidage (82) relié à la structure de référence (83) et adapté pour être disposé dans une deuxième position par rapport à la structure de référence (83), le deuxième élément de guidage (82) possédant une extrémité distale et une extrémité proximale et un deuxième passage conduisant à l'extrémité distale, le deuxième passage étant disposé, lorsque le premier élément de guidage (81) est introduit dans le premier trou, de façon à guider un perforateur d'os lors de la formation dans l'os d'un deuxième trou qui fait un angle avec et qui croise le premier trou ; **caractérisé par**
un dispositif récupérateur (21) disposé de façon à récupérer, à partir du passage du premier ou du deuxième élément de guidage (81, 82), un fil, une suture ou un autre élément inséré dans le passage de l'autre parmi le premier ou le deuxième élément de guidage (81, 82).

2. Appareil selon la revendication 1, dans lequel :
le premier et le deuxième élément de guidage (81, 82) comportent chacun une butée (811, 821) ; et
la structure de référence (83) est construite et disposée de façon à venir en prise avec les butées (811, 821) du premier et du deuxième élément de guidage (81, 82) de sorte que, lorsque les butées (811, 821) du premier et du deuxième élément de guidage (81, 82) sont en prise avec la structure de référence (83), les extrémités distales du premier et du deuxième élément de guidage (81, 82) sont adjacentes l'une à l'autre.

3. Appareil selon la revendication 1 ou la revendication 2, dans lequel les extrémités distales du premier et du deuxième élément de guidage (81, 82) sont adaptées pour être introduites à force dans l'os.

4. Appareil selon l'une quelconque des revendications 1 à 3, dans lequel le premier et le deuxième passage du premier et du deuxième élément de guidage (81, 82) s'étendent depuis les extrémités proximales du premier et du deuxième élément de guidage (81, 82) jusqu'à l'extrémité distale du premier et du deuxième élément de guidage (81, 82).

5. Appareil selon l'une quelconque des revendications 1 à 4, dans lequel le premier passage possède une section transversale dont la taille est inférieure à celle de la section transversale du deuxième passage.

6. Appareil selon l'une quelconque des revendications 1 à 5, comprenant en outre au moins un perforateur d'os (9) construit et disposé de façon à former un trou dans l'os tout en étant guidé en orientation par le passage du deuxième élément de guidage (82).

7. Appareil selon l'une quelconque des revendications 1 à 6, dans lequel le dispositif récupérateur (21) est disposé de façon à récupérer une suture depuis le passage du premier ou du deuxième élément de guidage (81, 82) et l'appareil comprend en outre un dispositif de fixation de suture construit et disposé de façon à être positionné par rapport à une voie de passage dans l'os et de façon à aider à immobiliser une suture positionnée au moins en partie dans la voie de passage.

8. Appareil selon l'une quelconque des revendications 1 à 7, dans lequel la structure de référence (83) et le premier et le deuxième élément de guidage (81, 82) sont construits et disposés de façon à être utilisés dans une intervention d'arthroscopie afin de former une voie de passage à travers l'os.

9. Appareil selon l'une quelconque des revendications 1 à 8, dans lequel la structure de référence (83) comporte un élément de liaison et un premier et un deuxième manchon (84, 85) comportant chacun un premier et un deuxième orifice et fixés à l'élément de liaison.

10. Appareil selon la revendication 9, dans lequel :
le premier et le deuxième manchon comportent chacun un alésage et une surface de prise ; et
le premier et le deuxième élément de guidage (81, 82) comportent chacun une butée (811, 821) ;
dans lequel le premier et le deuxième élément de guidage (81, 82) peuvent être reçus dans l'alésage du premier et du deuxième manchon, respectivement, de telle façon que les butées (811, 821) viennent en prise avec une surface de prise respective et positionnent les extrémités distales adjacentes l'une à l'autre.

11. Appareil selon la revendication 10, dans lequel le premier et le deuxième élément de guidage (81, 82) sont disposés en angle l'un par rapport à l'autre lorsqu'ils sont reçus dans leurs manchons respectifs.

12. Appareil selon la revendication 11, dans lequel l'angle est d'environ 90 degrés.

13. Appareil selon l'une quelconque des revendications 1 à 12, dans lequel le premier et le deuxième élément de guidage (81, 82) comportent chacun un tube construit et disposé de façon à être positionné dans une canule d'arthroscopie.

14. Appareil selon l'une quelconque des revendications 1 à 13, dans lequel l'appareil est disposé de façon à être utilisé avec une voie de passage dans un humérus afin d'effectuer une réparation de la coiffe des rotateurs.

15. Appareil selon l'une quelconque des revendications 1 à 16, dans lequel le premier et le deuxième élément de guidage (81, 82) et la structure de référence (83) sont construits et disposés de façon à coopérer avec une voie de passage dans un humérus, le premier élément de guidage (81) passant par un abord arthroscopique supéro-latéral et le deuxième élément de guidage (82) passant par un abord arthroscopique latéral, tandis que la structure de référence (83) est positionnée à l'extérieur du corps.
